# EUROPÄISCHE PATENTSCHRIFT

(11) **EP 1 171 105 B1**
(45) Veröffentlichungstag und Bekanntmachung des Hinweises auf die Patenterteilung: **14.07.2004**
(21) Anmeldenummer: 00925177.8
(22) Anmeldetag: 07.04.2000
(51) Int. Cl.: A61K 9/70, A61M 37/00, A61F 13/02, A61L 15/44

(54) **TRANSDERMALES THERAPEUTISCHES SYSTEM MIT EINEM STARK WIRKSAMEN NEUROLEPTIKUM**
TRANSDERMAL THERAPEUTIC SYSTEM WITH A HIGHLY EFFECTIVE NEUROLEPTIC AGENT
SYSTEME THERAPEUTIQUE TRANSDERMIQUE CONTENANT UN NEUROLEPTIQUE TRES ACTIF

(30) Priorität: 22.04.1999 DE 19918105
(43) Veröffentlichungstag der Anmeldung: 16.01.2002
(73) Patentinhaber: LTS Lohmann Therapie-Systeme AG, 56626 Andernach (DE)
(72) Erfinder: BRACHT, Stefan, D-56299 Ochtendung (DE); HORSTMANN, Michael, D-56564 Neuwied (DE)
(74) Vertreter: Flaccus, Rolf-Dieter, Dr.
(86) Internationale Anmeldenummer: PCT/EP2000/003113
(87) Internationale Veröffentlichungsnummer: WO 2000/064419

(56) Entgegenhaltungen:
- EP-A1- 0 156 080
- EP-A2- 0 452 837
- US-A- 5 882 676
- US-A- 5 891 461
- VOIGT R.: "Pharmazeutische Technologie f r Studium und Beruf" 1993, ULLSTEIN MOSBY GMBH & CO KG, BERLIN 7. Auflage, Seiten 399 - 400, Kapitel 16.2.1.2 und Seiten 591 - 596, Kapitel 26.4.3.3.5, XP002901174.

## Beschreibung

Fluphenazin ist ein tricyclisches, sehr stark wirksames Neuroleptikum aus der Gruppe der Perphenazine. Diese Substanzen besitzen eine antipsychotische Wirkung, - insbesondere bei schizophrenen Psychosen -, ohne das Bewußtsein und die intellektuellen Fähigkeiten wesentlich zu beeinflussen. Die typische orale Tagesdosis beträgt 3-6 mg, unter stationären Bedingungen bis zu 24 mg (vgl. Mutschler E. "Arzneimittelwirkungen", 6. Aufl., Wissenschaftliche Verlagsgesellschaft Stuttgart 1991).
Die Halbwertszeit im Blutplasma liegt bei 15 h. Für die intravenöse Therapie stehen die Esterformen, z.B. Decanoat und
Enanthat mit jeweils deutlich verlängerter Halbwertszeit zur Verfügung. In der oralen Therapie wird das Dihydrochlorid des Fluphenazins verwendet (vgl. Rote Liste Win 1997/II Vers. 2.4, ROTE LISTE Service GmbH, ECV Editio Cantor Verlag).

Die Therapie an schizophrenen Psychosen erkrankter Patienten erfordert typischerweise die chronische, oft lebenslange Verabreichung entsprechender Medikamente.
Die Patienten sind häufig nur bedingt oder nur zeitweise ansprechbar, so daß eine aktive Mitwirkung bei der Therapie häufig nicht zu erreichen ist. Eine selbständige Einnahme durch den Patienten ist folglich mit großen Unsicherheiten behaftet.

Es ist deshalb Gegenstand der Erfindung, ein fluphenazinhaltiges transdermales therapeutisches System (TTS) bereitzustellen, das mindestens 1 µg/cm²·d Wirkstoff an die menschliche Haut abgibt und damit die ein- oder sogar mehrmalige tägliche orale Einnahme durch eine 1-3 mal wöchentliche Applikation ersetzt.

Das Problem kann dadurch gelöst werden, daß das TTS einen Enhancer aufweist und eine hautseitige haftklebende Schicht auf der Basis von Polymeren besitzt, die reinen Kohlenwasserstoff darstellen.

Der Entwicklung entsprechender transdermaler therapeutischer Systeme stand bisher die Vermutung einer nur sehr geringen Hautgängigkeit für Fluphenazin und insbesondere seiner Salze gegenüber.
Für Fluphenazin-Dihydrochlorid ist daher wegen der Salzstruktur und der damit verbundenen Hydrophilie eine schlechte Permeabilität bei Humanhaut zu erwarten. Erschwerend kommen das relativ hohe Molekulargewicht von 437.53 Da sowie der sterisch fixierte Trizyklus im Molekül hinzu.

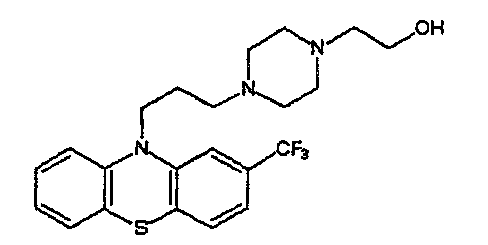

Die transdermale Aufnahme von mehreren Milligramm pro Tag, auf einer akzeptablen Applikationsfläche von max. 50 cm², stößt daher auf gewisse Vorbehalte.
Folglich finden sich in der Literatur keine Beschreibungen für praxisgerechte transdermale Systeme, die eine systemische Wirksamkeit erzielen.
Vielmehr findet sich Fluphenazin in der Patentliteratur nur an solchen Stellen erwähnt, wo TTS mit bestimmten physikochemischen Eigenschaften (US 5.474.783) oder Zusatzstoffen beschrieben werden (US 5.120.545), ohne daß ein Bezug zu konkreten Ausführungsbeispielen für diesen Wirkstoff hergestellt wird. Diese Patentschriften beinhalten Fluphenazin lediglich als einen möglichen Wirkstoff aus einer rein theoretisch erstellten Liste erdenklicher Wirkstoffe.

Beispielsweise offenbart EP 0 452 837 ein transdermales therapeutisches System in Pflasterform, welches aus einem Wirkstoff, einem hydrophoben Polymer mit einer Glasübergangstemperatur von -65 °C bis 35 °C, einem perkutanen Absorptionsverbesserer sowie einem hydrophilen Polymer, das wasserlöslich oder in Wasser quellfähig ist, besteht. Als Wirkstoff wird neben vielen weiteren Wirkstoffen Fluphenazin-HCl oder Flupentixol erwähnt, als hydrophobes Polymer sollen auch Polyisobutylene in Betracht kommen können, auch wenn (Meth) acrylat-Copolymere mit funktionalen Monomeren besonders bevorzugt werden. US 5.882.676 offenbart transdermale therapeutische Systeme, bei denen der Permeations-Enhancer Acyllactylat als essentieller Bestandteil neben dem Wirkstoff, u. a. Fluphenazin oder Triflupromazin, in einer Matrix, u. a. auf Polyisobutin-Basis, eingebettet ist. Jedoch offenbaren weder EP 0 452 837 noch US 5.882.676 konkrete Beispiele für transdermale therapeutische Systeme, die die vorliegend beanspruchten Wirkstoffe enthalten.

Kürzlich wurden nun neuere Untersuchungen zur Pharmakokinetik von Fluphenazin nach oraler Gabe veröffentlicht (Koytchev R et al.: "Absolute Bioavailability of oral immediate and slow release fluphenazine in healthy volunteers", Eur. J. Clin. Pharmacol. 1996;51: 183-187). Im Ergebnis werden nur etwa 2,5 bis 3,5 % der oral verabreichten Dosis von Fluphenazin im Blut verfügbar.
Die direkte Verabreichung in den Blutstrom unter Umgehung von verdauungstrakt und First-Pass-Effekt in der Leber, wie sie transdermal möglich ist, würde daher mit einem Bruchteil der typischen oralen Dosis auskommen.

Eine typische transdermale Tagesdosis sollte von 90 bis 180 µg, unter stationären Bedingungen bis zu 840 µg zu erwarten sein.

Alle Untersuchungen wurden mit Fluphenazin-Dihydrochlorid (ICN Biomedicals Inc. Ohio, USA) durchgeführt. Diese Substanzform wird weltweit therapeutisch eingesetzt, so daß im Gegensatz zur freien Base auf sehr umfangreiche toxikologische und regulatorische Dossiers zugegriffen werden kann. Die Untersuchung der Hautgängigkeit erfolgte in vitro unter Verwendung von Kuheuter-Vollhaut bzw. humaner Epidermis, die durch Hitzeseparierung von humaner Vollhaut getrennt wurde.
Die Versuche wurden bei 32°C in einer geeigneten Permeationsapparatur (modifizierte Franz-Zelle) durchgeführt und in den erhaltenen Proben Fluphenazin durch eine geeignete HPLC-Methode vermessen. Allen Wertangaben liegen n=3 Proben zugrunde.

Im Rahmen der Matrix- oder auch Drug-in-Adhesive-Technologie wurden zunächst Haftkleberfilme auf der Basis von Poly(meth)acrylaten als Matrices untersucht. Es handelte sich um die Marktprodukte Durotak 387-2051, Durotak 387-2287- und Durotak 387-2353 (National Starch and Chemical Co.).
Solche Haftkleber finden wegen ihrer hohen Bautfreundlichkeit und des geringen Allergisierungspotentials breite Anwendung in medizinischen Produkten.

Das Dihydrochlorid-Salz ist in solchen Polymeren bzw. den zur Verarbeitung erforderlichen organischen Lösungsmitteln nahezu unlöslich. Es wurde daher in allen Fällen ein Zusatz von Eudragit E100 (Röhm Pharma GmbH) vorgesehen. Dieses Poly(meth)-acrylatcopolymer aus neutralen Methacrylsäureestern und Dimethylaminoethylmethacrylat weist Trialkylaminogruppen in der Seitenkette auf und ist in der Lage, als Anionenaustauscherharz zu fungieren. In diesem Sinne werden die Chlorid-Ionen des Fluphenazin-Dihydrochlorids bei gleichzeitiger Aufnahme von Protonen an Eudragit E100 gebunden, wobei Fluphenazin als freie Base in einem gewissen Gleichgewicht gebildet wird.
Günstig ist ein mindestens äquimolares Verhältnis von Eudragit E100 und Fluphenazin-Dihydrochlorid, d. h. von Gewichtsmengen, die dasselbe Äquivalentgewicht als Kaliumhydroxid berechnet aufweisen. Die Menge des alkalischen Zusatzstoffes, vorzugsweise eines Polymers wie z. B. Eudragit E100, kann aber auch vorzugsweise dem 0,5 bis 1,5fachen Äquivalentgewicht der enthaltenen Wirkstoffmenge, ausgedrückt als Kaliumhydroxid, entsprechen.

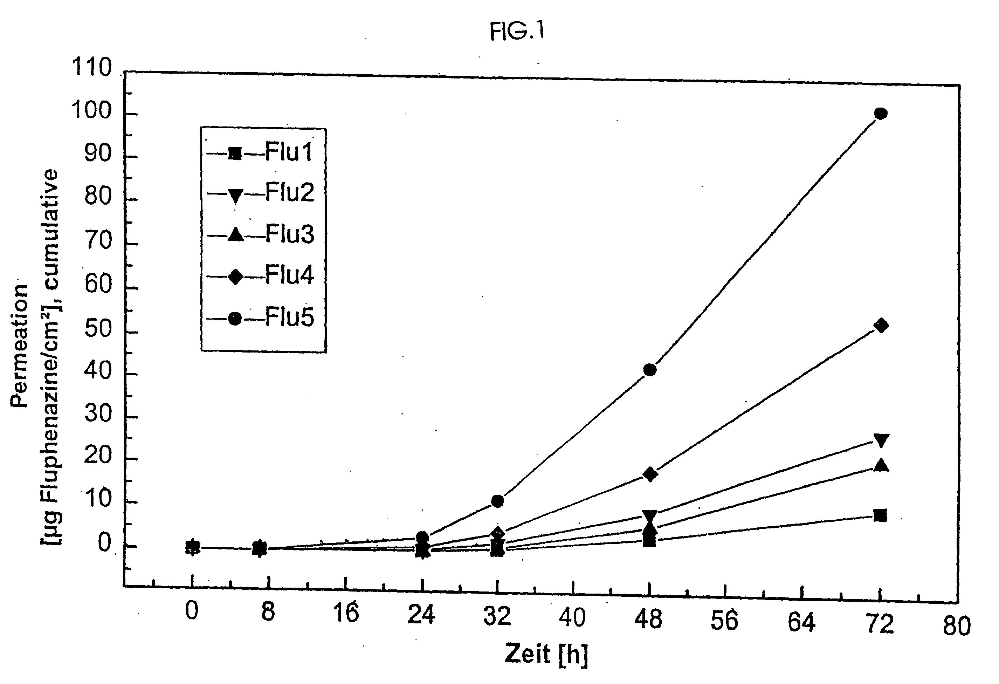

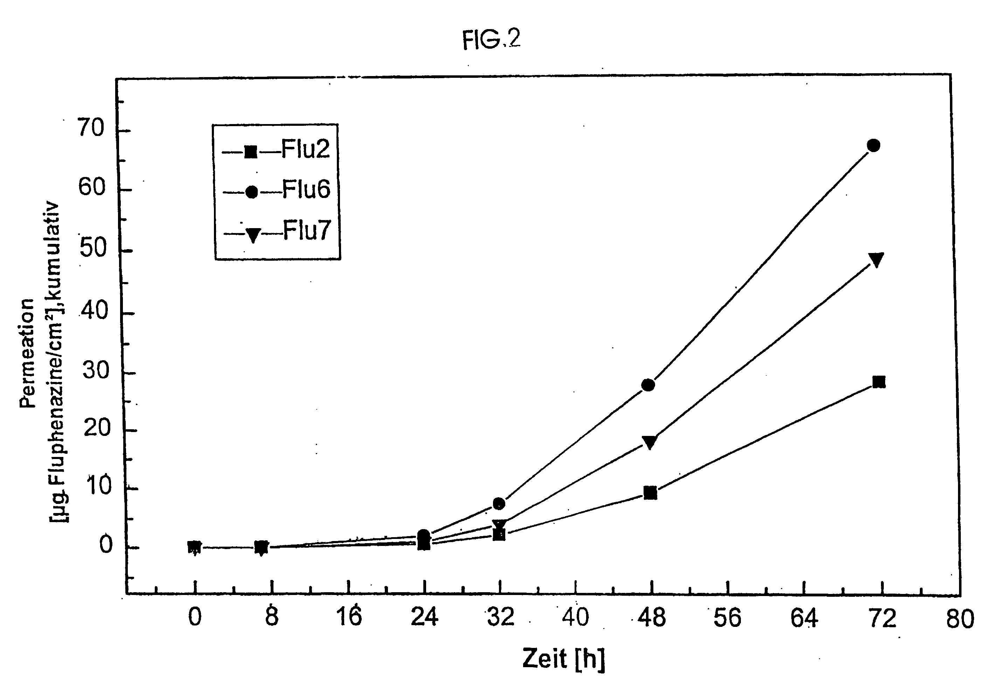

Bei dieser vorgehensweise wurde eine überraschend hohe Löslichkeit von mindestens 15 Gew.% Fluphenazin-Dihydrochlorid in Durotak 387-2051 gefunden, für Durotak 387-2287 noch mindestens 10 Gew.%.
Dennoch fielen die Permeationsraten an Kuheuterhaut nur sehr niedrig aus (Beispiele Flu1-Flu5, s. FIG.1, vgl. Tab.1).
Der carboxylgruppenfreie Durotak 387-2287 (vgl. Beispiel Flu5) erwies sich allerdings gegenüber dem carboxylgruppenhaltigen 387-2051 (vgl. Beispiele Flu3 und Flu5) als deutlich überlegen. Carboxylgruppen sind offenbar durch die Möglichkeit zur Salzbildung mit Fluphenazin-Base für dessen Freisetzung hinderlich. Diese Annahme konnte bestätigt werden, indem Durotak 387-2051 bei sonst gleichen Bedingungen mit einer äquimolaren Menge Kaliumhydroxid neutralisiert wurde (vgl. Beispiele Flu2 und Flu4). Dadurch ergaben sich erhöhte Premeationswerte, die allerdings dennoch nicht an das neutrale Durotak 387-2287 heranreichen.
Insgesamt ergibt sich für Klebermatrices auf Basis von Poly(meth)acrylat eine hohe Löslichkeit von Fluphenazin bei gleichzeitig schlechter Freisetzung.
Damit ist insbesondere der mengenmäßige Nutzungsgrad des enthaltenen Wirkstoffs sehr schlecht.
Dies konnte überraschenderweise durch Zusätze verbessert werden. Sowohl durch den Fettalkohol 2-Octyl-dodecanol (Eutanol® G) wie auch durch die Fettsäure Ölsäure konnte die Permeation durch Kuheuterhaut deutlich gesteigert werden (s. FIG.2, vgl. Tab.2).
Insbesondere die Fettsäure zeigt deutlich positive Effekte, die auf einer Ionenpaar-Bildung mit Fluphenazin-Base beruhen können. Die gute Hautgängigkeit solcher Ionen-Paar-Komplexe ist in der Fachwelt bekannt.

Während also die Verwendung eines basischen Hilfsstoffes wie Eudragit E100 bei gleichzeitiger Verwendung eines sauren Hilfsstoffes wie Ölsäure deutliche Vorteile erbringt, erscheinen Haftkleber auf Poly(meth)acrylatbasis als wenig geeignete Matrix.

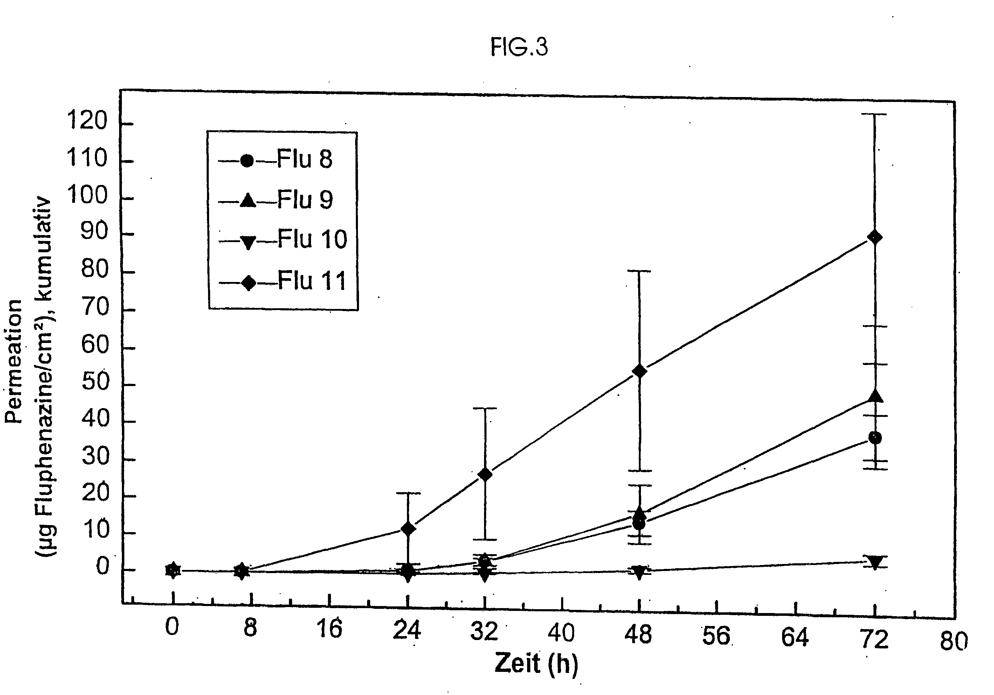

Das entwickelte Prinzip wurde daher auf andere mögliche Klebermatrices übertragen.
Überraschend wurde hierbei gefunden, daß eine Polymermatrix auf Basis reiner Kohlenwasserstoffe (Oppanol B10 und B100) zu sehr stark verbesserten Permeationsraten durch Kuheuterhaut führt (s. FIG.3, vgl. Tab.3).

Insbesondere in frühen Phasen des Versuches, nach 24 und 32 Stunden zeigt sich ein beträchtlicher Vorteil gegenüber vergleichsmatrices.
Die haftklebende Formulierung auf Basis reiner Kohlenwasserstoff-Polymere zeigt damit deutliche Vorteile gegenüber Poly(meth)acrylaten und überraschend auch gegenüber einem silikonkleber (BioPSA Q7-4301, Dow Corning Chem.Co.).

Vorzugsweise besteht die hautseitig haftklebende Schicht im wesentlichen aus Polymeren aus der Gruppe der Polyisobutylene oder der Polyisoprene . Gemäß einer besonderen Ausfuhrungsform ist vorgesehen, daß diese Schicht aus zwei bis drei verschiedenen Polymeren aufgebaut ist, die sich bei gleicher Molekularstruktur nur in ihren mittleren Molekulargewichten unterscheiden.

Speziell das Verhältnis von Wirkstoffbeladung (jetzt nur noch 5 Gew.-%, vorzugsweise zwischen 0,5 und 5 Gew.-% Wirkstoff in der Matrixschicht) zu Wirkstoffpermeation und damit der Nutzungsgrad haben sich erheblich verbessert.

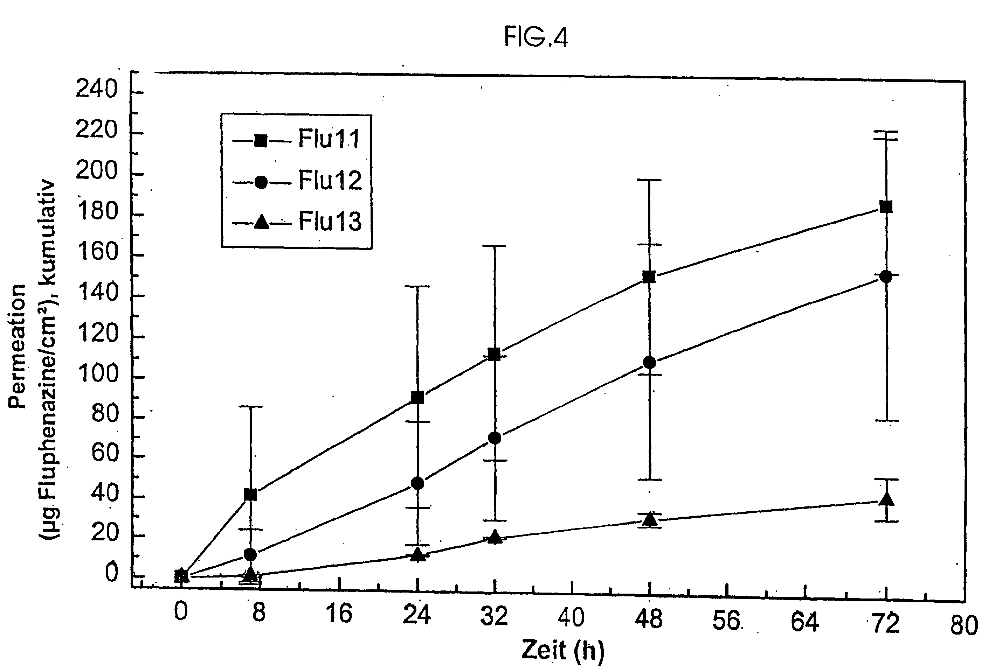

In einem weiteren Experiment wurde die optimierte Rezeptur schließlich auf humaner Epidermis getestet. Die erhaltenen Daten ergeben einen ausgezeichneten Permeationsverlauf mit kurzer Lagtime und annähernd linearer Charakteristik (s. FIG.4).
Als optimal erwies sich ein äquimolares Verhältnis von Fluphenazin, Eudragit E100 und ölsäure (bezogen auf die jeweiligen Äquivalentgewichte, berechnet als Kaliumhydroxid). Sowohl eine Erhöhung des Anteils der Ölsäure als auch des Eudragit E100 führten jeweils zu schlechteren Ergebnissen (s. FIG.4, vgl. Tab.4).

Es wurden maximale Flußraten von 2,9 µg/h·cm² Fluphenazin-Base erzielt.
Die zu erwartende transdermale Tagosdosis von 90-180 µg Fluphenazin (s.o.) könnte damit schon mit einem nur etwa 2-4 cm² großen TTS erzielt werden. Selbst die unter stationärer Therapie möglicherweise erforderlichen 840 µg täglich könnten transdermal mit einem System von weniger als 20 cm² Größe erreicht werden.

Auf Basis dieser Daten ist die transdermale Therapie mit Fluphenazin möglich geworden. Dabei sind im Rahmen der Erfindung sogar überraschend kleine TTS möglich.

Durch die Erfindung wird es möglich, die transdermale Therapie mit Fluphenazin bei einer Dosierung durchzuführen, die weit unterhalb der oral erforderlichen Mengen liegt.
Die transdermale Therapie mit Fluphenazin ist nicht nur eine alternative Verabreichungsform, sondern bietet sogar wegen ihrer größeren dosisbezogenen Effizienz Vorteile gegenüber der gängigen oralen Dauerthorapie.

Aufgrund der großen chemischen Ähnlichkeit, der gleichartigen pharmakodynamischen Wirkung, der vergleichbaren erforderlichen therapeutischen Dosierungen und der zu erwartenden Ähnlichkeit der Pharmakokinetik ist die Erfindung auf folgende weitere Wirkstoffe übertragbar:

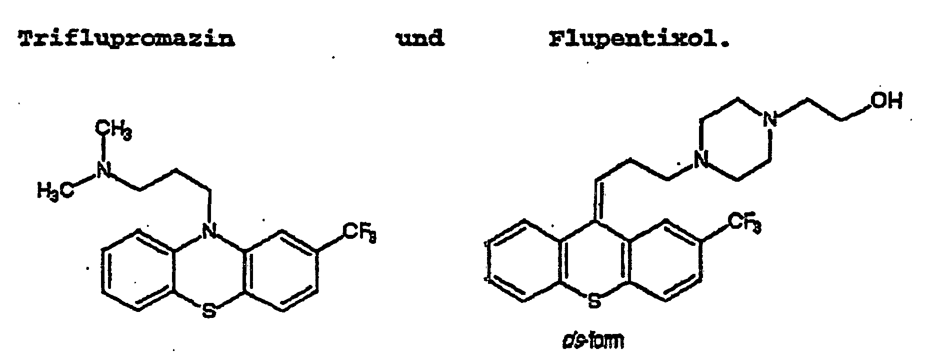

Bei Flupentixol ist das cis-Isomer (∝-Flupentixol) wegen seiner größeren pharmakodynamischen Potenz zu bevorzugen.

Bei dem enthaltenen Wirkstoff kann es sich auch um eine pharmazeutisch akzeptable Salzform handeln, vorzugsweise um die des Hydrochlorids oder des Dihydrochlorids.

Insbesondere erstreckt sich die Erfindung deshalb auf transdermale therapeutische Systeme, bestehend aus einer Rückschicht, mindestens einer wirkstoffhaltigen Matrixschicht, die gleichzeitig auch haftklebende Eigenschaften aufweisen kann, sowie einer ablösbaren Schutzschicht, wobei mit diesen Systemen eine Abgaberate von mindestens 1 µg/cm²·d eines Neuroleptikums, ausgewählt aus der Fluphenazin, Flupentixol und Triflupromazin umfassenden Gruppe, an die menschliche Haut erreicht wird.

Die Erfindung betrifft ferner ein Verfahren zur Verabreichung eines stark wirksamen Neuroleptikums an eine Person, welche die Behandlung mit einem solchen Wirkstoff benötigt, wobei der Wirkstoff Fluphenazin ist, und mit einer Rate von mindestens 1 µg/ cm²·d an die menschliche Haut abgegeben wird. Auf entsprechende Weise und mit der genannten Rate können mit einem solchen Verfahren auch die Wirkstoffe Flupentixol oder Triflupromazin an die menschliche Haut abgegeben werden.

Die erfindungsgemäßen transdermalen therapeutischen Systeme können somit zur Verabreichung eines stark wirksamen Neuroleptikums, ausgewählt aus der Fluphenazin, Flupentixol und Triflupromazin umfassenden Gruppe, an eine Person, welche die Behandlung mit einem solchen Wirkstoff benötigt, verwendet werden.

Das erfindungsgemäße Verfahren zur Verabreichung der genannten Neuroleptika und die Verwendung der erfindungsgemäßen TTS zur Verabreichung dieser Neuroleptika sind besonders vorteilhaft bei der Behandlung von Patienten, welche an Psychosen oder schizophrenen Psychosen erkrankt sind. Wie eingangs erwähnt wurde, ist bei speziell bei solchen Patienten, die meist über längere Zeit medikamentös behandelt werden müssen, die orale Verabreichung von Medikamenten mit Nachteilen verbunden.

Im folgenden sei auf weitere Anforderungen an ein TTS hingewiesen:

Wegen der bekannten Photoreaktivität des Phenothiazingerüstes können stabilisierende Zusätze nötig werden. Dabei handelt es sich neben UV-Strahlung absorbierenden Stoffen oder Pigmenten speziell um Antioxidantion. Dies sind vorzugsweise Ascorbylpalmitat, Vitamin E und seine pharmazeutisch akzeptablen Ester sowie Butylhydroxyanisol (BHA) und Butylhydroxytoluol (BHT). Es können auch schwefelhaltige Stabilisatoren wie Methionin oder anorganische Sulfite- erforderlich sein. Auch die Verwendung von Hexamethylentetramin (Methenamin) als spezifischer Stabilisator für Phenothiazine ist möglich (s. Monographie "Phenothiazin" in The Merck Index, 12^{th} Edition 1996).

Der Zusatz solcher Stoffe erfolgt typischerweise in Konzentrationen von unter 1 Gew.-%, vorzugsweise in einer Menge von 0,01 bis 1,0 Gew.-%, zu der wirkstoffhaltigen Matrix des TTS.

Im Hinblick auf die Lichtempfindlichkeit kann es weiterhin sinnvoll sein, als Rückschicht des TTS eine durch Pigmentierung, Lackierung oder Metallisierung lichtundurchdringlich gemachte Folie oder einen entsprechenden Materialverbund zu verwenden.

Ferner ist gemäß einer weiteren Ausführungsform vorgesehen, daß in den TTS ein Tackifier als Zusatzstoff enthalten ist, vorzugsweise aus der Gruppe von Mineralöl sowie von natürlichen oder synthetischen Harzen.

Bei dem erwähnten Permeations-Enhancer kann es sich vorzugsweise um eine gesättigte oder einfach ungesättigte Fettsäure der allgemeinen Formel H₂ₓ₊₁Cₓ-COOH bzw. H₂ₓ₋₁Cₓ-COOH für X=S bis 17, insbesondere um Undecylensäure, Laurinsäure, Myristinsäure oder Ölsäure handeln, wobei die Fettsäure in einer Menge entsprechend vorzugsweise dem 0,5 bis 1,5fachen Äquivalentgewicht der enthaltenen Wirkstoffmenge, berechnet als Kaliumhydroxid, zugesetzt wird.

Des weiteren kann es sich bei dem Permeations-Enhancer um einen gesättigten oder einfach ungesättigten Fettalkohol der allgemeinen Formel H₂ₓ₊₁Cₓ-CH₂-OH bzw. H₂ₓ₋₁Cₓ-CH₂-OH für X=5 bis 17, insbesondere um 1-Decanol, 1-Dodecanol, Oleylalkohol oder den verzweigtkettigen Alkohol 2-Octyl-dodecanol handeln, wobei dieser Enhancer in einer Menge von vorzugsweise 1 bis 20 Gew.-% in der Wirkstoff haltigen Matrixschicht enthalten ist.

Ferner kommen als Permeations-Enhancer bevorzugt Verbindungen aus der Gruppe der Fettalkoholpolyoatyethylether, der Fettsäuremethyloster, der Fottsäureothylester, der rettsäureisopropylester, der Fettsäurelactate oder der Fettalkoholfettsäureester in Betracht, wobei der Enhancer jeweils in einer Menge von vorzugsweise 1 bis 20 Gew.-% in der wirkstoffhaltigen Matrixschicht enthalten ist.

### Beispiele 1 bis 13:

Die Herstellung der Beispielrezepturen Flul bis Flu13 erfolgte unter den im folgenden geschilderten allgemeinen Bedingungen:
Die verschiedenen Durotak-Kleber und der Silikonkleber wurden in Form der vom Hersteller gelieferten Lösungen in organischen Lösungsmitteln eingesetzt.
Eudragit E100 wurde in Form einer Lösung in Ethylacetat (60 Gew.%) verarbeitet.
Die Mischung von 75 Gewichtsteilen Oppanol B10 mit 25 Gewichtsteilen Oppanol B100 wurde als Lösung in Spezialbenzin 80-110 verwendet (31 Gew.%).
Die Neutralisierung carboxylgruppenhaltiger Polyacrylatkleber (Durotak 387-2051 und 387-2353) erfolgte durch Umsetzung dieser Kleborlösungen mit Kaliumhydroxid in methanolischer Lösung (10 Gew.-%). Die eingesetzte Menge Kaliumhydroxid entsprach der Untergrenze der vom Hersteller spezifizierten Kaliumhydroxid-Zahl (mg KOH/g Polymer) für das jeweilige Produkt.

Die angegebene Menge Fluphenazin-Dihydrochlorid (Fluphenazin·2 HCl) wurde zunächst mit der Eudragit-Lösung vermischt, bevor die Haftkleberlösungen und schließlich gegebenenfalls weitere Bestandteile eingearbeitet wurden. Eine gegebenenfalls notwendige Verdünnung der Masse auf eine geeignete Viskosität erfolgte mit Ethylacetat.
Die homogen gerührten Massen wurden mit einem Balkenauftragswerk auf eine 100 µm starke Folie aus silikonisiertem Polyethylentherephtalat (PET) beschichtet und anschließend 5 Minuten bei 80°C in einem Abluftofen getrocknet.
Der getrocknete Kleberfilm wurde mit einer PET-Folie (15 µm Dicke) als Schutzfolie abgedeckt.
Das Flächengewicht der Klebermatrix wurde durch geeignete Wahl der Beschichtungsstärke in allen Fällen auf 80 g/m² eingestellt.

Die hier tabellarisch wiedergegebenen Zusammensetzungen der Beispielrezepturen beziehen sich auf die getrocknete wirkstoffhaltige Schicht des TTS (Tab.1 bis 4).

**Tab.1:**

| Zusammensetzung der haftklebenden Matrixschicht in Gewichtsprozent: | | | | | |
|---|---|---|---|---|---|
| Bestandteile | Beispiel Flu1 | Beispiel Flu2 | Beispiel Flu3 | Beispiel Flu4 | Beispiel Flu5 |
| Fluphenazin 2 HCl | 11,7 | 11,7 | 17,6 | 17,6 | 11,7 |
| Eudragit E100 | 14,3 | 14,3 | 21,5 | 21,5 | 14,3 |
| Durotak 387-2051 | 74,0 | --- | 39,1 | --- | --- |
| Durotak 387-2051 Kalium-Salz* | --- | 74,0 | --- | 39,1 | --- |
| Durotak 387-2287 | --- | --- | --- | --- | 74,0 |
| Total | 100,0 | 100,0 | 100,0 | 100,0 | 100,0 |

**Tab.2:**

| Zusammensetzung der haftklebenden Matrixschicht in Gewichtsprozent: | | |
|---|---|---|
| Bestandteile | Beispiel Flu6 | Beispiel Flu7 |
| Fluphenazin 2 HCl | 11,7 | 11,7 |
| Eudragit E100 | 14,3 | 14,3 |
| Ölsäure | 6,5 | --- |
| Eutanol G | --- | 5,0 |
| Durotak 387-2051 Kalium-Salz* | 67,5 | 69,0 |
| Total | 100,0 | 100,0 |

**Tab.3:**

| Zusammensetzung der haftklebenden Matrixschicht in Gewichtsprozent: | | | | |
|---|---|---|---|---|
| Bestandteile | Beispiel Flu8 | Beispiel Flu9 | Beispiel Flu10 | Beispiel Flu11 |
| Fluphenazin 2 HCl | 5,83 | 5,83 | 5,83 | 5,83 |
| Eudragit E100 | 7,15 | 7,15 | 7,15 | 7,15 |
| Ölsäure | 3,22 | 3,22 | 3,22 | 3,22 |
| Eutanol G | --- | --- | 20,0 | --- |
| Bio PSA Q7-4301 | 83,8 | --- | --- | --- |
| Durotak 387-2287 | --- | 83,8 | 63,8 | --- |
| Durotak 387-2353 Kalium-Salz* | --- | --- | --- | --- |
| Oppanol B10/B100 75+25* | --- | --- | --- | 83,8 |
| Total | 100,0 | 100,0 | 100,0 | 100,0 |

**Tab.4:**

| Zusammensetzung der haftklebenden Matrixschicht in Gewichtsprozent: | | |
|---|---|---|
| Bestandteile | Beispiel Flu12 | Beispiel Flu13 |
| Fluphenazin2 HCl | 5,83 | 5,83 |
| Eudragit E100 | 10,7 | 7,15 |
| Ölsäure | 3,22 | 4,83 |
| OppanolB10/B100 75+25* | 80,3 | 82,2 |
| Total | 100,0 | 100,0 |

## Patentansprüche

1. Transdermales therapeutisches System, bestehend aus einer Rückschicht, mindestens einer wirkstoffhaltigen Matrixschicht, die gleichzeitig auch haftklebende Eigenschaften aufweisen kann, sowie einer ablösbaren Schutzschicht, **gekennzeichnet durch** einen Gehalt eines Neuroleptikums, einen Gehalt mindestens eines Permeations-Enhancers sowie eine hautseitig haftklebende Schicht auf der Basis von Polymeren, die reine Kohlenwasserstoffe darstellen.

2. Transdermales therapeutisches System nach Anspruch 1, **dadurch gekennzeichnet, daß** das neuroloptikum Fluphenazin, Flupentixol oder Triflupromazin ist.

3. Transdermales therapeutisches System nach Anspruch 1 oder 2, **dadurch gekennzeichnet, daß** die Wirkstoffkonzentration in der Matrixschicht zwischen 0,5 und 5,0 Gew.% beträgt.

4. Transdermales therapeutisches System nach einem oder mehreren der Ansprüche 1 bis 3, **dadurch gekennzeichnet, daß** die Abgaberate des Neuroleptikums mindestens 1 µg/cm²·d beträgt.

5. Transdermales therapeutisches System nach einem oder mehreren der Ansprüche 1 bis 4, **dadurch gekennzeichnet, daß** es sich bei dem enthaltenen Wirkstoff um eine pharmazeutisch akzeptable Salzform, vorzugsweise die des Hydrochlorides oder des Dihydrochlorides handelt.

6. Transdermales therapeutisches System nach einem oder mehreren der vorangehenden Ansprüche, **dadurch gekennzeichnet, daß** ein alkalisch reagierender Zusatzstoff enthalten ist, vorzugsweise in einer Menge, die dem 0,5 bis 1,5fachen Äquivalentgewicht der enthaltenen Wirkstoffmenge, ausgedrückt als Kaliumhydroxid, entspricht.

7. Transdermales therapeutisches System nach einem oder mehreren der vorangehenden Ansprüche, **dadurch gekennzeichnet, daß** es sich bei dam alkalischen Zusatzstoff um ein Polymer, vorzugweise um ein Copolymer aus Dimethylaminoethylmethacrylat- und Methacrylat-Einheiten handelt.

8. Transdermales therapeutisches System nach Anspruch 1, **dadurch gekennzeichnet, daß** es sich bei dem Permeations-Enhancer um eine gesättigte oder einfach ungesättigte Fettsäure der allgemeinen Formel H₂ₓ₊₁Cₓ-COOH bzw. H₂ₓ₋₁Cₓ-COOH für X=5 bis 17, insbesondere um Undecylensäure, Laurinsäure, Myristinsäure oder Ölsäure in einer Menge von vorzugsweise dem 0,5 bis 1, 5fachen Äquivalentgewicht der enthaltenen Wirkstoffmenge, berechnet als Kaliumhydroxid, handelt.

9. Transdermales therapeutisches System nach Anspruch 1, **dadurch gekennzeichnet, daß** es sich bei dem Permeations-Enhancer um einen gesättigten oder einfach ungesättigten Fettalkohol der allgemeinen Formel H₂ₓ₊₁Cₓ-CH₂-OH bzw. H₂ₓ₋₁Cₓ-CH₂-OH für X=5 bis 17, insbesondere um 1-Decanol, 1-Dodecanol, Oleylalkohol oder den verzweigtkettigen Alkohol 2-Octyl-dodecanol in einer Menge von vorzugsweise 1 bis 20 Gew.%, bezogen auf die Wirkstoffhaltige Matrixschicht, handelt.

10. Transdermales therapeutisches System nach Anspruch 1, **dadurch gekennzeichnet, daß** es sich bei dem Permeations-Enhancer um eine Verbindung aus der Gruppe der Fettalkoholpolyoxyethylether, der Fettsäuremethylester, der Fettsäureethylester, der Fettsäureisopropylester, der Fettsäurelactate oder der Fettalkoholfettsäureester in einer Menge von vorzugsweise 1 bis 20 Gew.%, bezogen auf die wirkstoffhaltige Matrixschicht, handelt.

11. Transdermales therapeutisches System nach Anspruch 1, **dadurch gekennzeichnet, daß** die hautseitig haftklobende Schicht im wesentlichen aus Polymeren aus der Gruppe der Polyisobutylene oder der Polyisoprene besteht.

12. Transdermales therapeutisches System nach Anspruch 1 und 10, **dadurch gekennzeichnet, daß** die hautseitig haftklebende Schicht aus zwei bis drei verschiedenen Polymeren aufgebaut ist, die sich bei gleicher Molekularstruktur nur in ihren mittleren Molekulargewichten unterscheiden.

13. Transdermales therapeutisches System nach Anspruch 1, **dadurch gekennzeichet, daß** als Zusatzstoff ein Tackifier, vorzugsweise aus der Gruppe von Mineralöl sowie von natürlichen oder synthetischen Harzen enthalten ist.

14. Transdermales therapeutisches System nach einem oder mehreren der vorangehenden Ansprüche, **dadurch gekennzeichnet, daß** ein stabilisierender Zusatz aus der Gruppe von Antioxidantien oder Hexamothylentetramin in einer Menge von vorzugsweise 0,01 bis 1,0 Gew.% in der haftklebenden Schicht enthalten ist.

15. Transdermales therapeutisches System mit einem Gehalt an einem Neuroleptikum aus der Fluphenazin, Flupentixol und Triflupromazin umfassenden Gruppe, bestehend aus einer Rückschicht, mindestens einer wirkstoffhaltigen Matrixschicht, die gleichzeitig auch haftklebende Eigenschaften aufweisen kann, sowie einer ablösbaren Schutzschicht, **dadurch gekennzeichnet, daß** die Matrixschicht eine Polymerschicht auf der Basis von reinen Kohlenwasserstoffen ist und mindestens einen Permeations-Enhancer enthält, und daß es das Neuroleptikum mit einer Abgaberate von mindestens 1 µg/cm²·d an die menschliche Haut abgibt.

## Claims

1. Transdermal therapeutic system consisting of a backing layer, at least one active substance-containing matrix layer, which may at the same time possess pressure-sensitive adhesive properties, as well as a removable protective layer, **characterized by** a content of a neuroleptic, a content of at least one permeation enhancer, as well as by a layer which is pressure-sensitive adhesive on the skin-facing side and based on polymers which are pure hydrocarbons.

2. Transdermal therapeutic system according to Claim 1, **characterized in that** the neuroleptic is fluphenazine, flupentixol or triflupromazine.

3. Transdermal therapeutic system according to Claim 1 or 2, **characterized in that** the active substance concentration in the matrix layer is between 0.5 and 5.0%-wt.

4. Transdermal therapeutic system according to one or more of Claims 1 to 3, **characterized in that** the release rate of the neuroleptic is at least 1 µg/cm²·d.

5. Transdermal therapeutic system according to one or more of Claims 1 to 4, **characterized in that** the active substance contained is a pharmaceutically acceptable salt form, preferably that of the hydrochloride or the dihydrochloride.

6. Transdermal therapeutic system according to one or more of the preceding Claims, **characterized in that** an alkaline-reacting additive is contained, preferably in an amount corresponding to 0.5 to 1.5 times the equivalent weight of the active substance amount contained, expressed as potassium hydroxide.

7. Transdermal therapeutic system according to one or more of the preceding claims **characterized in that** the alkaline additive is a polymer, preferably a copolymer of dimethylaminoethyl methacrylate and methacrylate units.

8. Transdermal therapeutic system according to Claim 1, **characterized in that** the permeation enhancer is a saturated or monounsaturated fatty acid of the general formula H₂ₓ₊₁Cₓ-COOH and H₂ₓ₋Cₓ-COOH, respectively, for X = 5 to 17, especially undecylenic acid, lauric acid, myristic acid or oleic acid, in an amount of preferably 0.5 to 1.5 times the equivalent weight of the active substance amount contained, calculated as potassium hydroxide.

9. Transdermal therapeutic system according to Claim 1, **characterized in that** the permeation enhancer is a saturated or monounsaturated fatty alcohol of the general formula H₂ₓ₊₁Cₓ-CH₂-OH and H₂ₓ₋₁Cₓ-CH₂-OH, respectively, for X = 5 to 17, especially 1-decanol, 1-dodecanol, oleyl alcohol or the branched-chain alcohol 2-octyl dodecanol, in an amount of preferably 1 to 20%-wt. relative to the active substance-containing matrix layer.

10. Transdermal therapeutic system according to Claim 1, **characterized in that** the permeation enhancer is a compound from the group of fatty alcohol polyoxyethyl ethers, the fatty acid methyl esters, the fatty acid ethyl esters, the fatty acid isopropyl esters, the fatty acid lactates or the fatty alcohol fatty acid esters in an amount of preferably 1 to 20%-wt., relative to the active substance-containing matrix layer.

11. Transdermal therapeutic system according to Claim 1, **characterized in that in that** the layer which is pressure-sensitive adhesive on the skin-facing side substantially consists of polymers from the group of polyisobutylenes or polyisoprenes.

12. Transdermal therapeutic system according to Claims 1, and 10, **characterized in that** the layer which is pressure-sensitive adhesive on the skin-facing side is made up of two to three different polymers which having the same molecular structure differ only in their mean molecular weight.

13. Transdermal therapeutic system according to Claim 1, **characterized in that** as active substance a tackifier, preferably from the group of mineral oils as well as of natural or synthetic resins, is contained.

14. Transdermal therapeutic system according to one or more of the preceding Claims, **characterized in that** a stabilizing additive from the group of antioxidants or hexamethylenetetramine is contained in an amount of preferably 0.01 to 1.0%-wt. in the pressure-sensitive adhesive layer.

15. Transdermal therapeutic system with a content of a neuroleptic of the group comprising fluphenazine, flupentixol and triflupromazine, consisting of a backing layer, at least one active substance-containing matrix layer, which may at the same time possess pressure-sensitive adhesive properties, as well as a removable protective layer, **characterized in that** the matrix layer is a polymer layer based on pure hydrocarbons and contains at least one permeation enhancer, and that the said transdermal therapeutic system releases the neuroleptic to the human skin at a release rate of at least 1 µg/cm²·d.

## Revendications

1. Système thérapeutique transdermique constitué par une couche dorsale, par au moins une couche matricielle contenant une substance active, qui peut également présenter de manière simultanée des propriétés autoadhésives, ainsi que par une couche de protection détachable, **caractérisé par** une teneur en un neuroleptique, par une teneur en au moins un agent favorisant la perméation, et par une couche autoadhésive du côté tourné vers la peau, à base de polymères qui représentent des hydrocarbures purs.

2. Système thérapeutique transdermique selon la revendication 1, **caractérisé en ce que** le neuroleptique est la fluphénazine, le flupentixol ou la triflupromazine.

3. Système thérapeutique transdermique selon la revendication 1 ou 2, **caractérisé en ce que** la concentration de la substance active dans la couche matricielle se situe entre 0,5 et 5,0 % en poids.

4. Système thérapeutique transdermique selon une ou plusieurs des revendications 1 à 3, **caractérisé en ce que** le taux de libération du neuroleptique s'élève à au moins 1 µg/cm²·d.

5. Système thérapeutique transdermique selon une ou plusieurs des revendications 1 à 4, **caractérisé en ce que**, en ce qui concerne la substance active qu'il contient, il s'agit d'une forme de sel pharmaceutiquement acceptable, de préférence de celle du chlorhydrate ou du dichlorhydrate.

6. Système thérapeutique transdermique selon une ou plusieurs des revendications précédentes, **caractérisé en ce qu'**il contient un additif réagissant de manière alcaline de préférence en une quantité qui représente de 0,5 à 1,5 fois le poids équivalent de la quantité de substance active qu'il contient, exprimée comme hydroxyde de potassium.

7. Système thérapeutique transdermique selon une ou plusieurs des revendications précédentes, **caractérisé en ce que**, en ce qui concerne l'additif alcalin, il s'agit d'un polymère, de préférence d'un copolymère constitué par des unités de diméthylaminoéthylméthacrylate et de méthacrylate.

8. Système thérapeutique transdermique selon la revendication 1, **caractérisé en ce que**, en ce qui concerne l'agent favorisant la perméation, il s'agit d'un acide gras saturé ou simplement insaturé répondant à la formule générale H₂ₓ₊₁CₓCOOH, respectivement H₂ₓ₋₁Cₓ-COOH pour x = de 5 à 17, en particulier de l'acide undécylénique, de l'acide laurique, de l'acide myristique ou de l'acide oléique en une quantité qui représente de préférence de 0,5 à 1,5 fois le poids équivalent de la quantité de substance active qu'il contient, calculée comme hydroxyde de potassium.

9. Système thérapeutique transdermique selon la revendication 1, **caractérisé en ce que**, en ce qui concerne l'agent favorisant la perméation, il s'agit d'un alcool gras saturé ou simplement insaturé répondant à la formule générale H₂ₓ₊₁Cₓ-CH₂-OH, respectivement H₂ₓ₋₁Cₓ-CH₂-OH pour x = de 5 à 17, en particulier du 1-décanol, du 1-dodécanol, de l'alcool oléylique ou de l'alcool à chaîne ramifiée, le 2-octyl-dodécanol, en une quantité de préférence de 1 à 20 % en poids, rapportés à la couche matricielle contenant la substance active.

10. Système thérapeutique transdermique selon la revendication 1, **caractérisé en ce que**, en ce qui concerne l'agent favorisant la perméation, il s'agit d'un composé choisi parmi le groupe des éthers polyoxyéthyliques d'alcools gras, des esters méthyliques d'acides gras, des esters éthyliques d'acides gras, des esters isopropyliques d'acides gras, des lactates d'acides gras ou des esters d'alcools gras-acides gras, en une quantité de préférence de 1 à 20 % en poids rapportés à la couche matricielle contenant la substance active.

11. Système thérapeutique transdermique selon la revendication 1, **caractérisé en ce que** la couche adhésive du côté tourné vers la peau est constituée essentiellement par des polymères choisis parmi le groupe des polyisobutylènes ou des polyisoprènes.

12. Système thérapeutique transdermique selon les revendications 1 et 10, **caractérisé en ce que** la couche adhésive du côté tourné vers la peau est composée par deux à trois polymères différents qui, tout en possédant la même structure moléculaire, ne se distinguent que par leurs poids moléculaires moyens.

13. Système thérapeutique transdermique selon la revendication 1, **caractérisé en ce qu'**il contient, à titre d'additif, un agent poisseux, de préférence choisi parmi le groupe comprenant de l'huile minérale et des résines naturelles ou synthétiques.

14. Système thérapeutique transdermique selon une ou plusieurs des revendications précédentes, **caractérisé en ce qu'**il contient, un additif de stabilisation choisi parmi le groupe comprenant des antioxydants, ou de l'hexaméthylènetétramine en une quantité de préférence de 0,01 à 1,0 % en poids, dans la couche autoadhésive.

15. Système thérapeutique transdermique possédant une teneur en un neuroleptique choisi parmi le groupe comprenant la fluphénazine, le flupentixol et la triflupromazine, constitué par une couche dorsale, par au moins une couche matricielle contenant une substance active, qui peut également présenter de manière simultanée des propriétés autoadhésives, ainsi que par une couche de protection détachable, **caractérisé en ce que** la couche matricielle est une couche polymère à base d'hydrocarbures purs et contient au moins un agent favorisant la perméation, et **en ce qu'**il libère le neuroleptique sur la peau humaine avec un taux de libération d'au moins 1 µg/cm²·d.
